(19) Europäisches Patentamt
European Patent Office
Office européen des brevets

(11) Veröffentlichungsnummer: **0 153 636**
**B1**

(12) **EUROPÄISCHE PATENTSCHRIFT**

(45) Veröffentlichungstag der Patentschrift: **06.12.89**

(51) Int. Cl.⁴: **C 07 F 17/02,** A 61 K 31/28

(21) Anmeldenummer: **85101370.6**

(22) Anmeldetag: **08.02.85**

# (54) Metalliceniumsalze und deren Verwendung als Cytostatica bei der Krebsbekämpfung.

(30) Priorität: **08.02.84 DE 3404443**

(43) Veröffentlichungstag der Anmeldung:
**04.09.85 Patentblatt 85/36**

(45) Bekanntmachung des Hinweises auf die Patenterteilung:
**06.12.89 Patentblatt 89/49**

(84) Benannte Vertragsstaaten:
**AT BE CH DE FR GB IT LI LU NL SE**

(56) Entgegenhaltungen:
**CHEMISCHE BERICHTE, Band 93, 1960, Weinheim, DE, A.N. NESMEJANOW et al.: "Unmittelbare Cyanierung der Ferriciniumsalze", pp. 2729-2735**
**JOURNAL OF ORGANOMETALLIC CHEMISTRY, Band 60 (1973), pp. C17,C18, Elsevier Sequoia S.A. (Lausanne, CH), M. CASTAGNOLA et al.: "The electron donor properties of ferrocene. The oxidation of ferrocene by carboxylic acids"**
**CHEMICAL COMMUNICATIONS, 1965, London, GB, M. ALY et al.: "Paramagnetic ferrocene acid adducts. Kinetics of electron transfer to proton acids", pp. 404-406**

(73) Patentinhaber: **Köpf- Maier, Petra, Prof. Dr., Bundesring 33, D-1000 Berlin 42 (DE)**
Patentinhaber **Köpf, Hartmut, Prof. Dr., Bundesring 33, D-1000 Berlin 42 (DE)**
Patentinhaber: **Neuse, Eberhard Wilhelm, Prof. Dr., 5 Neeron Road, Blairgowrie- Randburg 2194 (ZA)**

(72) Erfinder: **Köpf- Maier, Petra, Prof. Dr., Bundesring 33, D-1000 Berlin 42 (DE)**
Erfinder: **Köpf, Hartmut, Prof. Dr., Bundesring 33, D-1000 Berlin 42 (DE)**
Erfinder: **Neuse, Eberhard Wilhelm, Prof. Dr., 5 Neeron Road, Blairgowrie- Randburg 2194 (ZA)**

(74) Vertreter: **Barz, Peter, Dr., Patentanwälte Dr. V. Schmied- Kowarzik Dipl.- Ing. G. Dannenberg Dr. P. Weinhold Dr. D. Gudel Dipl.- Ing. S. Schubert Dr. P. Barz Siegfriedstrasse 8, D-8000 München 40 (DE)**

**EP 0 153 636 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Metallicenium-Verbindungen entstehen bei der chemischen oder elektrochemischen Einelektronen-Oxidation von Metallocenen; siehe Wilkinson et al., J. Am. Chem. Soc., Bd. (1952) 74, S. 2125 und Rosenblum, "Chemistry of the Iron Group Metallocenes", Wiley, New York (1965). Sie stellen salzartige Substanzen dar, die zum Teil hydrophil oder sogar wasserlöslich sind. In ihnen ist der Cyclopentadienyl Ring über eine hapto-5-Bindung mit dem Zentralmetallatom verknüpft.

Es wurde nun gefunden, daß Metallicenium-Verbindungen chemotherapeutisch wirksam sind und insbesondere cytostatische Eigenschaften aufweisen.

Gegenstand der Erfindung sind Metallicenium-Salze der allgemeinen Formel I

$$[(\eta^5\text{-}C_{5\text{-}x}R_x)M(\eta^5\text{-}C_5H_{5\text{-}y}R'_y)]^{m+}{}_a\,[A]^{n-}{}_b \qquad (I)$$

in der M Fe, Co oder Ru ist;

$C_5H_{5-x}R_x$ und $C_5H_{5-y}R'_y$ Cyclopentadienyl-Ringe bedeuten, die unabhängig voneinander unsubstituiert (x, y = 0) oder ein- bis fünffach substituiert sein können (x, y = 1, 2, 3, 4 oder 5), wobei R und R' unabhängig voneinander für gleiche oder verschiedene Substituenten stehen und Alkyl, Cycloalkyl, Hydroxyalkyl, Aminoalkyl, Halogenalkyl, Alkenyl, Aryl, Aralkyl, Ferrocenyl, Ferrocenylium, Ferrocenylalkyl, Acyl, Halogenyl, Trialkylsilyl, Tricycloalkylsilyl, Triarylsilyl, Trialkylsilyl oder Carboxyl, Ester-, Amid-oder Hydrazid-Gruppen bedeuten oder R und R' zusammen eine Alkylen-Brücken mit 2 bis 4 Brückenatomen bilden;

wobei die Alkylgruppen 1 bis 10 Kohlenstoffatome, die Cycloalkylgruppen 3 bis 8 Kohlenstoffatome, die Alkenylgruppen 2 bis 10 Kohlenstoffatome, die Arylgruppen 6 bis 18 Kohlenstoffatome und die Acylgruppen bis zu 12 Kohlenstoffatome enthalten;

a, b, m und n ganze Zahlen sind, wobei m den Wert 1 oder 2 und n den Wert 1, 2 oder 3 hat und $a \cdot m = b \cdot n$; und A ein komplexbildendes oder stark raumerfüllendes, das Metallicenium-Kation stabilisierendes Anion ist; und Solvate dieser Salze zur Verwendung als Arzneistoffe, insbesondere ihre Verwendung als Cytostatica bei der Krebsbekämpfung.

Eine bevorzugte Gruppe von erfindungsgemäß verwendeten Verbindungen sind die preisgünstig verfügbaren Ferricenium- und Cobalticenium-Verbindungen (M = Fe oder Co); aus dem gleichen Grund sind unsubstituierte Verbindungen (x, y = 0) bevorzugt.

Wenn R oder R' Alkyl, Hydroxyalkyl, Aminoalkyl, Halogenalkyl oder Halogenyl sind, haben x oder y vorzugsweise den Wert 1, 2, 3, 4 oder 5. Wenn R oder R' Cycloalkyl, Alkenyl, Aryl, Aralkyl, Ferrocenyl, Ferrocenylium, Ferrocenylalkyl, Acyl, Trialkylsilyl, Tricycloalkylsilyl, Triarylsilyl, Triaralkylsilyl oder eine Carboxyl-, Ester-, Amid-oder Hydrazid-Gruppe sind, haben x oder y vorzugsweise den Wert 1 oder 2.

Unter "Alkyl" werden geradkettige oder verzweigte Alkylreste mit 1 bis 10, vorzugsweise 1 bis 6 und insbesondere 1 bis 4 Kohlenstoffatomen verstanden. Spezielle Beispiele sind Methyl, Ethyl, Isopropyl, n-Butyl, tert.-Butyl, 2-Ethyl-hexyl und n-Decyl.

Unter "Cycloalkyl" werden cycloaliphatische Reste mit 3 bis 8, vorzugsweise 3 bis 6 und insbesondere 6 Kohlenstoffatomen verstanden. Spezielle Beispiele sind Cyclopropyl, Cyclobutyl, Cyclohexyl, Cycloheptyl und Cyclooctyl.

Unter "Alkenyl" werden ungesättigte Reste mit 2 bis 10, vorzugsweise 2 bis 6 und insbesondere 2 bis 4 Kohlenstoffatomen verstanden. Ein spezielles Beispiel ist Vinyl.

Unter "Aryl" werden aromatische und kondensiert-aromatische Reste mit 6 bis 18, vorzugsweise 6 bis 14 und insbesondere 6 bis 10 Kohlenstoffatomen verstanden. Ein spezielles Beispiel ist Phenyl.

Unter "Halogen" werden Fluor, Chlor, Brom und Jod verstanden, wobei Fluor, Chlor und Brom bevorzugt sind.

Unter "Acyl" werden aliphatische oder aromatische Acylreste mit bis zu 12, vorzugsweise bis zu 7 und insbesondere bis zu 4 Kohlenstoffatomen verstanden. Spezielle Beispiele sind Acetyl, Propionyl und Benzoyl.

Die Hydroxyalkyl-, Aminoalkyl-, Halogenalkyl-, Alkylen-, Aralkyl-, Ferrocenylalkyl-, Trialkylsilyl-, Tricycloalkylsilyl-, Triarylsilyl- und Triaralkylsilyl-Substituenten leiten sich vorzugsweise von den genannten Alkyl-, Cycloalkyl-, Aryl- bzw. Halogenresten ab. Die Estergruppen sind vorzugsweise Alkylestergruppen mit 1 bis 4 Kohlenstoffatomen im Alkylrest.

Bevorzugte Beispiele für das Anion A sind anionische Komplexe des Typs $[M'X_4]^{n-}$ (n = 1 oder 2) oder $[M'X_6]^{n-}$ (n = 1, 2 oder 3), wobei M' = B, Bi, Co, Fe, Ga, Hg, Ru oder Sb und X = F, Cl, Br oder Phenyl. Hiervon sind besonders bevorzugt $BF_4^-$ $B\varnothing_4^-$, $FeCl_4^-$, $FeBr_4^-$, $BiCl_4^-$, $GaCl_4^-$, $PF_6^-$ und $RuC_4$.

Weitere bevorzugte Anionen A sind Polyhalogenide, wie $Br_3^-$ und $J_3$ Vanadate, Molybdate, Wolframate, Oxyvanadate, Heteropolyanionen, wie Molybdophosphate, Wolframatophosphate und Wolframatosilikate, Reineckate, Hexafluorophosphate, Chinonide, Chinondimethanide, $(Cl_3Fe\text{-}O\text{-}FeCl_3)^{2-}$ und Anionen starker organischer Säuren, wie Mono-, Di- oder Trihalogenessigsäure, Cyanessigsäure, Perchlorsäure und Pikrinsäure.

Die Metallicenium-Salze können gegebenenfalls solvatisiert sein, z. B. durch 1, 2 oder 3 Mol der dem Anion A entsprechenden freien Säure.

Repräsentative Beispiele für erfindungsgemäß verwendbare Metallicenium-Salze sind:

Ferricenium-tetrachloroferrat
1,1'-Bis(triphenylsilyl)ferricenium-tetrachloroferrat
Ferricenium-tetrabromoferrat
Ruthenicenium-tetrachlororuthenat
Ferricenium-tetrafluoroborat

Acetylferricenium-tetrafluoroborat
Biferricenium-tetrafluoroborat
Biferricenium-bis(tetrafluoroborat)
1'1'''-Diethylbiferricenium-bis(tetrafluoroborat)
Ferrocenylmethyl-ferrocenylium-tetrafluoroborat
Ferricenium-tetraphenylborat
Ferricenium-tetrachloroantimonat
Ferricenium-hexachloroantimonat
Ferricenium-tetrachlorwismutat
Ferricenium-tetrachlorogallat
Ferricenium-hexafluorophosphat
1,1'-Dimethylferricenium-hexafluorophosphat
Decamethylferricenium-hexafluorophosphat
1,1'-Trimethylenferricenium-hexafluorophosphat
Cobalticenium-hexafluorophosphat
1,1'-Dimethylcobalticenium-hexafluorophosphat
Ferricenium-2,3-dichlor-5,6-dicyanhydrochinonid
Decamethylferricenium-2,3-dichlor-5,6-dicyanhydrochinonid
Cobalticenium-2,3-dichlor-5,6-dicyanhydrochinonid
Ferricenium-tetracyanchinodimethanid-tetracyanchinodimethan
1,1'-Dimethylferricenium-tetracyanchinodimethanid-tetracyanchinodimethan
Cobalticenium-tetracyanchinodimethanid-tetracyanchinodimethan
Ferricenium-perchlorat
1,1'-Trimethylenferricenium-perchlorat
Cobalticenium-perchlorat
Ferricenium-trichloracetat-bis(trichloressigsäure)
Ferricenium-trichloracetat-mono(trichloressigsäure)
1,1'-Dimethylferricenium-trichloracetat-bis(trichloressigsäure)
Biferricenium-trichloracetat-bis(trichloressigsäure)
Ferricenium-pikrat
Ferricenium-pikrat-pikrinsäure
Biferricenium-pikrat
Ferricenium-trijodid
Hydroxymethylferricenium-trijodid
Biferricenium-trijodid
Ferricenium-reineckat
Ferricenium-wolframatosilikat
Ferricenium-oxytrichlorvanadat
Ferricenium-metawolfram Diferricenium-μ-oxo-bis(trichloroferrat)

Bei diesen Verbindungen handelt es sich größtenteils um bekannte Verbindungen, die nach Literaturvorschriften oder verbesserten Verfahren, die in den nachstehenden Ausführungsbeispielen beschrieben sind, hergestellt werden können. Einige erfindungsgemäß verwendbare Metallicenium-Salze sind neu, z. B. Diferricenium-μ-oxo-bis(trichloroferrat). Die Herstellung dieser neuen Verbindungen ist ebenfalls in den Beispielen erläutert.

**Beispiel 1**

Ferricenium-tetrachloroferrat kann nach der von Nesmeyanov et al., Chem. Ber., Bd. 93, (1960) S. 2729 beschriebenen Methode aus Ferrocen und Eisen(III)-chlorid im Molverhältnis 1 : 2 in Ether hergestellt werden, wobei allerdings das Endprodukt selbst bei mehrfachem Umkristallisieren aus absolutem Ethanol mit Diferricenium-μ-oxo-bis(trichloroferrat) verunreinigt bleibt. Die genannte Verunreinigung zeigt sich durch Infrarotabsorption bei 365 und 316 $cm^{-1}$ an.

Es wurde nun gefunden, daß diese Verunreinigung quantitativ entfernt werden kann, indem man das rohe Tetrachloroferrat aus saurem, chloridionen-haltigem Medium einmal umkristallisiert:

Unreines Ferricenium-tetrachloroferrat wird nach dem Verfahren von Nesmeyanov et al. aus Ferrocen (2 mMol) und wasserfreiem Eisen(III)-chlorid (4 mMol) in Ether und Umkristallisieren aus absolutem Ethanol hergestellt. Das erhaltene Produkt, durch das Infrarotspektrum als ein mit Diferricenium-μ-oxo-bis(trichloroferrat) verunreinigtes Präparat ausgewiesen, läßt sich entweder aus frisch destilliertem Thionylchlorid oder aus 0,15 - 0,25 H wässrigmethanolischem (2 : 98) Chlorwasserstoff umkristallisieren, wobei ein spektroskopisch (Verschwinden der Maxima bei 365 und 316 $cm^{-1}$) und elementaranalytisch reines Ferriceniumtetrachloroferrat in Form von blauschwarzen Kristallblättchen erhalten wird. Gef.: C, 31.22; H, 2.71; Cl, 36.80. Ber. für $C_{10}H_{10}Cl_4Fe_2$: C, 31.30; H, 2.63; Cl, 36.96. Das Umkristallisieren aus dem sauren Medium kann auch direkt mit dem rohen, aus Ferrocen und Eisenchlorid erhaltenen Produkt unter Umgehung des Umkristallisierens aus absolutem Ethanol durchgeführt werden.

**Beispiel 2**

Ferricenium-trichloracetat-mono(trichloressigsäure) ist zwar in der Literatur (Castagnola et al., JOM, Bd. 60, C17 (1973)) beschrieben worden, läßt sich jedoch nicht reproduzierbar nach der Methode dieser Autoren aus Ferrocen und Trichloressigsäure im Molverhältnis 1 : 3 in benzolischer Lösung herstellen. Diese Vorschrift führt vielmehr hauptsächlich oder vollständig zur Bildung des Disolvates: Ferricenium-trichloracetat-bis(trichloressigsäure).

Es wurde nun gefunden, daß sich das Monosolvat leicht aus dem Disolvat durch hydrolytische Abspaltung eines Äquivalents Trichloressigsäure erhalten läßt, z. B. durch vorsichtiges Umkristallisieren aus Wasser:

Das als Ausgangsmaterial benötigte Disolvat wird nach einer der Vorschriften von Hendrickson et al., J. Chem. Phys. Bd. 58, (1973) S. 4666, hergestellt. Eine Lösung von 1,0 g Ferricenium-trichloracetat-bis(trichloressigsäure) in 9,0 ml Wasser wird 10 bis 15 Minuten am Sieden gehalten und noch heiß filtriert. Beim Abkühlen scheiden sich bereits dunkelblaue Kristallnadeln ab, die nach mehrstündigem Stehen abfiltriert, mit wenig Eiswasser gewaschen und bei 60°C / 27 Pa (0,2 Torr) getrocknet werden; Fp. 125 - 127 C. Eine weitere Portion des Salzes, Fp. 127 - 129°C, läßt sich nach starkem Einengen der Mutterlauge (mit den wässrigen Waschlösungen vereinigt), kurzem Aufwärmen zur Auflösung von bereits ausgeschiedenen Kristallen und Stehenlassen bei 50°C erhalten. Die Gesamtausbeuten liegen bei 0,4 - 0,6 g. Gef.: C, 32.78; H, 2.19; Cl, 41.89. Ber. für $C_{14}H_{11}Cl_6FeO_4$: C, 32.85; H, 2.17; Cl, 41.56. Das Salz löst sich leicht in Wasser und weniger leicht in organischen Lösungsmitteln, wie Methanol oder Acetonitril.

**Beispiel 3**

Diferricenium-µ-oxo-bis(trichloroferrat) ist zwar literaturbekannt (Aharoni und Litt, JOM, Bd. 22, (1970) S. 179, doch wurde dieser Verbindung von den Autoren irrtümlich die Struktur eines Ferricenium-trichloroferrats zugeschrieben. Darüberhinaus hat sich die Herstellungsvorschrift dieser Autoren als nicht reproduzierbar erwiesen.

Es wurde nun gefunden, daß das Salz sich leicht aus dem in Beispiel 2 beschriebenen Ferricenium-tetrachloroferrat durch Umkristallisieren aus mit Pyridin versetztem Methanol Acetonitril erhalten läßt, wobei die organische Base als Protonenfänger dient:

Eine Lösung von 2,0 g Ferricenium-tetrachloroferrat, erhalten gemäß Beispiel 2, in 15 ml einer $N_2$-gesättigten Mischung von Methanol/Acetonitril (1 : 1) wird heiß filtriert und dann mit einer Lösung von 225 mg Pyridin in 8 ml Ether versetzt. Die nach mehrstündigem Stehen bei -20°C im verschlossenen Gefäß abgeschiedenen bläulich-schwarzen Kristalle des Oxobis(trichloroferrates) werden abfiltriert, mit Ether gewaschen und bei 75°C / 27 Pa (0,2 Torr) getrocknet. Eine weitere Portion des Salzes wird nach Einengen der Mutterlauge im Rotationsverdampfer auf etwa ein Drittel des ursprünglichen Volumens und Behandeln wie zuvor gewonnen, und eine dritte, unreine Portion des Salzes, aus den weiter eingeengten Schlußlaugen erhalten, kann durch Umkristallisieren aus dem gleichen Lösungsmittelgemisch in reiner Form gewonnen werden. Gesamtausbeute 0,9 - 1,1 g. Gef.: C, 33.41, H, 2.83; Cl, 30.44; O, 2.35; Ber. für $C_{20}H_{20}Cl_6Fe_4O$: C, 33.71; H, 2.83; Cl, 29.85; O, 2.25. Die Verbindung ist gut löslich in Wasser und weniger leicht löslich in organischen Lösungsmitteln, wie Acetonitril oder Methanol.

**Beispiel 4**

Ferricenium-pikrat-pikrinsäure ist zwar in der Literatur erwähnt worden (Aly et al., Chem. Commun., 404 (1965)), jedoch ohne Angabe einer Darstellungsvorschrift.

Es wurde nun gefunden, daß sich das Salz durch Oxidation von Ferrocen in konzentrierter Schwefelsäure und nachfolgende Behandlung der mit Wasser verdünnten Ferriceniumsulfat-Lösung mit Pikrinsäure erhalten läßt:

Eine Lösung von 4,65 g (25 mMol) Ferrocen in 14 ml konz. $H_2SO_4$ wird nach halbstündigem Stehen mit 100 ml Eiswasser verdünnt und filtriert. Man gibt zum Filtrat eine heiße Lösung von 6,87 g (30 mMol) Pikrinsäure in 100 ml Wasser und läßt die Mischung 24 Stunden bei Raumtemperatur stehen. Der gebildete kristalline Niederschlag wird abfiltriert, mit wenig Wasser und Ether gewaschen und bei 50°C / 27 Pa (0,2 Torr) getrocknet. Man erhält 5,1 g Rohprodukt als grünschwarze Kristalle, die vorsichtig ohne längeres Erhitzen aus Ethanol umkristallisiert werden, wobei Ferricenium-pikrat-pikrinsäure in Form von schwärzlichen Nadeln, Fp. 128 - 130°C, gewonnen wird. Gef.: C, 41.99; H, 2.40; H, 12.30. Ber. für $C_{22}H_{15}FeN_6O_{14}$: C, 41.08; H, 2.35; H, 13.06. Das Salz ist leicht löslich in Wasser und weniger leicht löslich in organischen Lösungsmitteln, wie Alkoholen oder Acetonitril.

Die erfindungsgemäßen Metallicenium-Salze zeigen im Tierversuch cytostatische Wirkung. Die Testung erfolgt hierbei folgendermaßen:

Weibliche $CF_1$-Mäuse erhalten mittels intraperitonealer Injektion je etwa $6.10^6$ Ehrlich-Aszites-Tumorzellen und 24 Stunden später eine einmalige intraperitoneale Substanzgabe (Dosisbereich 20 bis 500 mg/kg) in physiologischer Kochsalzlösung (0,4 ml). Es werden jeweils 5 bis 10 Tiere pro Dosis getestet. Gegebenenfalls kann auf einen pH-Wert von 4 bis 7 gepuffert werden, z. B. mit Natriumhydrogen-carbonat oder Tris-(hydroxymethyl)-aminomethan, um eine lokale Irriation am Injektionsort zu vermeiden. In jeder Versuchsreihe wird eine Gruppe von unbehandelten Kontrolltieren, denen 0,4 ml physiologische Kochsalzlösung ohne Substanzgabe intraperitoneal injiziert wurde, mitgeführt.

Die Beurteilung der Tumorentwicklung in den einzelnen Dosisbereichen erfolgt anhand von Gewichtsverlauf und Überlebensdauer. Bei jeder Substanz werden die dosisabhängige Anzahl von Tumortodesfällen, Toxizitätstodesfällen und überlebenden, geheilten Tieren sowie die zugehörige prozentuale Zunahme der mittleren Oberlebensdauer bestimmt.

Die bei der Testung von Ferricenium-trichloracetat-mono(trichloressigsäure), Ferricenium-trichloracetat-bis(trichloressigsäure), Ferricenium-pikrat und Diferricenium-µ-oxo-bis(trichloroferrat) erzielten Ergebnisse sind in den folgenden Tabellen I bis IV wiedergegeben.

**Tab. I**

Wirkung von $Cp_2Fe^+CCl_3COO^- \cdot CCl_3COOH$ (*) auf die Überlebenszeit von Ehrlich-Aszites-Tumor-tragenden Mäusen

| Dosis [mg/kg] | Anzahl der Versuchstiere | Überlebende Tiere (a) Anzahl | Überlebende Tiere (a) Anteil [%] | Mittlere Überlebensdauer (a) [d] | Zunahme der mittleren Überlebensdauer (a) (b) [%] | |
|---|---|---|---|---|---|---|
| 20 | 6 | 1 | 17 | 27,3 | + | 72,8 |
| 40 | 6 | 1 | 17 | 28,3 | + | 79,1 |
| 60 | 6 | 3 | 50 | 50,3 | + | 218,4 |
| 80 | 6 | 4 | 67 | 64,0 | + | 305,1 |
| 100 | 6 | 3 | 50 | 58,3 | + | 269,0 |
| 120 | 6 | 5 | 83 | 76,8 | + | 386,1 |
| 140 | 6 | 5 | 83 | 76,4 | + | 383,5 |
| 160 | 6 | 5 | 83 | 75,7 | + | 379,1 |
| 180 | 6 | 4 | 67 | 61,3 | + | 288,0 |
| 200 | 6 | 4 | 67 | 61,0 | + | 286,1 |
| 220 | 6 | 2 | 33 | 32,5 | + | 105,7 |
| 240 | 6 | 1 | 17 | 17,8 | + | 12,7 |
| 260 | 6 | 0 | 0 | 3,3 | - | 79,1 |

(a) Bis zum Stichtag (90. Tag nach Transplantation).
(b) Bezogen auf die unbehandelten Kontrolltiere (mittlere Überlebenszeit 15,8 d).
(*) Cp = Cyclopentadienyl $C_5H_5$

**Tab.II**

Wirkung von $Cp_2Fe^+ CCl_3COO^- \cdot 2 CCl_3COOH$ (*) auf die Überlebenszeit von Ehrlich-Aszites-Tumor-tragenden Mäusen

| Dosis [mg/kg] | Anzahl der Versuchstiere | Überlebende Tiere (a) Anzahl | Überlebende Tiere (a) Anteil [%] | Mittlere Überlebensdauer (a) [d] | Zunahme der mittleren Überlebensdauer (a) (b) [%] | |
|---|---|---|---|---|---|---|
| 20 | 6 | 0 | 0 | 15,5 | + | 1,3 |
| 40 | 6 | 0 | 0 | 15,3 | ± | 0,0 |
| 60 | 6 | 0 | 0 | 15,8 | + | 3,3 |
| 80 | 6 | 0 | 0 | 16,2 | + | 5,9 |
| 100 | 6 | 1 | 17 | 25,3 | + | 65,4 |
| 120 | 6 | 2 | 33 | 38,5 | + | 151,6 |
| 140 | 6 | 2 | 33 | 38,7 | + | 152,9 |
| 160 | 6 | 4 | 67 | 65,0 | + | 324,8 |
| 180 | 6 | 4 | 67 | 66,3 | + | 333,3 |
| 200 | 6 | 5 | 83 | 78,0 | + | 409,8 |
| 220 | 6 | 6 | 100 | 90,0 | + | 488,2 |
| 240 | 6 | 6 | 100 | 90,0 | + | 488,2 |
| 260 | 6 | 6 | 100 | 90,0 | + | 488,2 |
| 280 | 6 | 6 | 100 | 90,0 | + | 488,2 |
| 300 | 6 | 6 | 100 | 90,0 | + | 488,2 |
| 320 | 6 | 5 | 83 | 76,0 | + | 396,7 |
| 340 | 6 | 6 | 100 | 90,0 | + | 488,2 |
| 360 | 6 | 4 | 67 | 61,2 | + | 300,0 |
| 380 | 6 | 3 | 50 | 47,0 | + | 207,2 |
| 400 | 6 | 4 | 67 | 61,8 | + | 303,9 |
| 420 | 6 | 2 | 33 | 33,0 | + | 115,7 |
| 440 | 6 | 1 | 17 | 18,3 | + | 19,6 |
| 460 | 6 | 2 | 33 | 32,5 | + | 112,4 |
| 480 | 6 | 0 | 0 | 3,5 | - | 77,1 |
| 500 | 6 | 0 | 0 | 3,2 | - | 79,1 |

(a) Bis zum Stichtag (90. Tag nach Transplantation)
(b) Bezogen auf die unbehandelten Kontrolltiere (mittlere Überbenzeit 15,2 d)
(*) Cp = Cyclopentadienyl $C_5H_5$

**Tab. III**

Wirkung von $Cp_2Fe^+$ $C_6H_2N_3O_7^-$ (*) auf die Überlebenszeit von Ehrlich-Aszites-Tumor-tragenden Mäusen

| Dosis [mg/kg] | Anzahl der Versuchstiere | Überlebende Tiere (a) Anzahl | Überlebende Tiere (a) Anteil [%] | Mittlere Überlebensdauer (a) [d] | Zunahme der mittleren Überlebensdauer (a) (b) [%] |
|---|---|---|---|---|---|
| 20 | 6 | 0 | 0 | 14,2 | - 2,7 |
| 40 | 6 | 0 | 0 | 14,5 | - 0,7 |
| 60 | 6 | 0 | 0 | 14,5 | - 0,7 |
| 80 | 6 | 0 | 0 | 15,7 | + 7,5 |
| 100 | 6 | 1 | 17 | 28,3 | + 93,8 |
| 120 | 6 | 1 | 17 | 28,0 | + 91,8 |
| 140 | 6 | 3 | 50 | 52,5 | + 259,6 |
| 160 | 6 | 2 | 33 | 40,3 | + 176,0 |
| 180 | 6 | 3 | 50 | 50,0 | + 242,5 |
| 200 | 6 | 5 | 83 | 78,0 | + 434,2 |
| 220 | 6 | 6 | 100 | 90,0 | + 516,4 |
| 240 | 6 | 6 | 100 | 90,0 | + 516,4 |
| 260 | 6 | 5 | 83 | 75,5 | + 417,1 |
| 280 | 6 | 6 | 100 | 90,0 | + 516,4 |
| 300 | 6 | 3 | 50 | 48,7 | + 233,6 |
| 320 | 6 | 4 | 67 | 62,0 | + 324,7 |
| 340 | 6 | 2 | 33 | 35,2 | + 141,1 |
| 360 | 6 | 3 | 50 | 48,0 | + 228,8 |
| 380 | 6 | 1 | 17 | 19,0 | + 30,1 |
| 400 | 6 | 2 | 33 | 34,0 | + 132,9 |
| 420 | 6 | 0 | 0 | 4,5 | - 69,2 |

(a) Bis zum Stichtag (90. Tag nach Transplantation)
(b) Bezogen auf die unbehandelten Kontrolltiere (mittlere Überlebenszeit 14,6 d)
(*) Cp = Cyclopentadienyl $C_5H_5$; $C_6H_2N_3O_7^-$ = Pikrat

**Tab. IV**

Wirkung von $(Cp_2Fe^+)_2$ $(Cl_3Fe\text{-}O\text{-}FeCl_3)^2$ (*) auf die Überlebenszeit von Ehrlich-Aszites-Tumor-tragenden Mäusen

| Dosis [mg/kg] | Anzahl der Versuchstiere | Überlebende Tiere (a) Anzahl | Überlebende Tiere (a) Anteil [%] | Mittlere Überlebensdauer (a) [d] | Zunahme der mittleren Überlebensdauer (a) (b) [%] |
|---|---|---|---|---|---|
| 20 | 6 | 1 | 17 | 28,7 | + 78,3 |
| 40 | 6 | 2 | 33 | 40,0 | + 148,4 |
| 60 | 6 | 1 | 17 | 27,8 | + 72,7 |
| 80 | 6 | 3 | 50 | 52,8 | + 228,0 |
| 100 | 6 | 2 | 33 | 40,7 | + 152,8 |
| 120 | 6 | 3 | 50 | 53,3 | + 231,1 |
| 140 | 6 | 4 | 67 | 65,0 | + 303,7 |
| 160 | 6 | 4 | 67 | 63,8 | + 296,3 |
| 180 | 6 | 5 | 83 | 77,7 | + 382,6 |
| 200 | 6 | 4 | 67 | 62,2 | + 286,3 |
| 220 | 6 | 4 | 67 | 63,5 | + 294,4 |
| 240 | 6 | 4 | 67 | 63,7 | + 295,7 |
| 260 | 6 | 3 | 50 | 48,2 | + 199,4 |
| 280 | 6 | 3 | 50 | 48,2 | + 199,4 |
| 300 | 6 | 1 | 17 | 18,3 | + 13,7 |

(a) Bis zum Stichtag (90. Tag nach Transplantation)
(b) Bezogen auf die unbehandelten Kontrolltiere (mittlere Überlebenszeit 16,1 d)
(*) Cp = Cyclopentadienyl $C_5H_5$

Zur Krebsbekämpfung können die erfindungsgemäßen Metallicenium-Salze als solche oder als Arzneimittel eingesetzt werden, die mindestens ein Metallicenium-Salz der allgemeinen Formel I neben pharmazeutisch verträglichen Träger-, Verdünnungs- und/oder Hilfsstoffen enthalten. Die pharmazeutische Zubereitung der Wirkstoffe erfolgt vorzugsweise in Form von Einheitsdosen, die auf die jeweilige Applikationsart abgestimmt sind. Eine Einheitsdosis kann z. B. eine Tablette, eine Kapsel, ein Suppositorium oder eine abgemessene Volumenmenge eines Pulvers, eines Granulats oder eine Lösung oder Suspension sein. Unter "Einheitsdosis" wird eine physikalisch bestimmte Einheit verstanden, die eine individuelle Menge des Wirkstoffes in Mischung mit einem geeigneten pharmazeutischen Träger-, Verdünnungs- und/oder Hilfsstoff enthält. Dabei wird die Wirkstoffmenge so gewählt, daß eine oder mehrere Einheiten üblicherweise für eine einzelne therapeutische Verabreichung ausreichen. Die Einheitsdosis kann auch unterteilbar sein, z. B. in Form von gekerbten Tabletten, wenn für eine einzelne therapeutische Verabreichung nur ein Bruchteil, z. B. eine Hälfte oder ein Viertel, der unterteilbaren Einheit benötigt wird. Die Arzneimittel der Erfindung enthalten, wenn sie in Einheitsdosis vorliegen, 1 bis 10 000 mg, vorzugsweise 5 bis 7 500 mg, Wirkstoff.

Die Arzneimittel der Erfindung werden vorzugsweise oral, rectal oder parenteral, z. B. intravenös, subcutan, intramuskulär, intrapleural, intraperitoneal, intrafokal oder perifokal, angewandt. Die therapeutische Verabreichung kann mittels Infusion kontinuierlich über mehrere Stunden oder durch ein- bis mehrmalige Einzelgaben oder Einzelinjektionen erfolgen. Die Verabreichungsfolge und die verabreichte Dosis können in Abhängigkeit von Natur und Stadium der Erkrankung sowie abhängig vom Behandlungsregime, insbesondere von Anzahl und Dosierungshöhe verabreichter Kombinationspräparate, stark variieren. Beispielsweise- kann eine Anfangsbehandlung mit täglich 200 bis 800 mg i.v. oder mit Einzeldosen, z. B. 10 bis 40 mg/kg i.v., in entsprechenden Intervallen und eine darauffolgende Dauerbehandlung mit 1 bis 4 Tabletten zu je 50 mg Wirkstoff erfolgen.

Die Arzneimittel bestehen in der Regel aus den erfindungsgemäßen Wirkstoffen und nicht-toxischen, pharmazeutisch annehmbaren Arzneimittelträgern, die als Zumischung in fester, halbfester oder flüssiger Form oder als Umhüllungsmittel, z. B. in Form einer Kapsel, eines Tablettenüberzuges, eines Beutels oder eines anderen Behältnisses für den Wirkstoff in Anwendung kommen. Der Träger kann hierbei z. B. als Vermittler für die Arzneimittelaufnahme durch den Körper, als Formulierungshilfsmittel, Süßungsmittel, Geschmacksmittel, Farbstoff oder Konservierungsmittel dienen.

Für die orale Verabreichung eignen sich z. B. Tabletten, Dragées, Hart- und Weichgelatinekapseln, dispergierbare Pulver, Granulate, wässrige und ölige Suspensionen, Emulsionen, Lösungen und Sirupe.

Tabletten können inerte Verdünnungsmittel, wie Calciumcarbonat, Calciumphosphat, Natriumphosphat oder Lactose; Granulierungs- und Verteilungsmittel, wie Maisstärke oder Alginate Bindemittel, wie Stärke, Gelatine oder Akaziengummi; und Gleitmittel, wie Aluminium- oder Magnesiumstearat, Talcum oder Siliconöl, enthalten. Gegebenenfalls sind die Tabletten mit einem Überzug versehen, der auch so beschaffen sein kann, daß er eine verzögerte Auflösung und Resorption des Arzneimittels im Gastrointestinaltrakt und damit z. B. eine bessere Verträglichkeit oder eine längere Wirkungsdauer bewirkt.

Gelatinekapseln können den Wirkstoff im Gemisch mit einem festen (z. B. Calciumcarbonat oder Kaolin) oder öligen (z. B. Oliven-, Erdnuß- oder Paraffinöl) Verdünnungsmittel enthalten.

Als Suspendiermittel eignen sich z. B Natriumcarboxymethylcellulose, Methylcellulose, Hydroxypropylcellulose, Natriumalginat, Polyvinylpyrrolidon, Traganthgummi oder Akaziengummi; als Dispergier- und Benetzungsmittel z. B. Polyoxyethylenstearat, Heptadecaethylenoxycetanol, Polyoxyethylensorbitmonooleat, Polyoxyethylensorbitanmonooleat oder Lecithin; als Konservierungsmittel z. B. Methyl- oder Propyl-hydroxybenzoat; als Geschmacks- und Süßungsmittel z. B. Saccharose, Lactose, Dextrose oder Invertzuckersirup.

Ölige Suspensionen können z. B. Erdnuß-, Oliven-, Sesam-, Kokos- oder Paraffinöl sowie Verdickungsmittel, wie Bienenwachs, Hartparaffin oder Cetylalkohol, Süßungsmittel, Geschmacksmittel und/oder Antioxidantien enthalten.

In Wasser dispergierbare Pulver und Granulate enthalten den Wirkstoff im Gemisch mit Dispergier-, Benetzungs- und Suspendiermitteln, z. B. den vorstehend genannten Substanzen und/oder Dimethylsulfoxid, sowie mit Süßungsmitteln, Geschmacksmitteln und/oder Farbstoffen.

Emulsionen können z. B. Oliven-, Erdnuß- oder Paraffinöl neben Emulgatoren, wie Akaziengummi, Traganthgummi, Phosphatiden, Sorbitanmonooleat oder Polyoxyethylensorbitanmonooleat, Süßungsmittel und/oder Geschmacksmitteln enthalten.

Zur rectalen Anwendung eignen sich Suppositorien, die mit Hilfe von bei Rektaltemperatur schmelzenden Bindemitteln hergestellt werden, z. B. Kakaobutter oder Polyethylenglykolen.

Parenteral können die Arzneimittel als sterile isotonische Kochsalzlösungen oder sonstige Lösungen angewandt werden. Um eine gleichmäßige Lösung bzw. Suspendierung zu erzielen, kann ein Lösungsvermittler, wie Dimethylsulfoxid, zugesetzt werden, jedoch ist dies meist nicht erforderlich.

In allen Darreichungsformen können die Arzneimittel der Erfindung außerdem Puffersubstanzen enthalten, z. B. Natriumhydrogencarbonat oder Tris(hydroxymethyl)aminomethan.

Neben den erfindungsgemäßen Metallicenium-Salzen können die Arzneimittel einen oder mehrere andere pharmakologisch aktive Bestandteile aus anderen cytostatisch wirksamen Arzneimittelgruppen enthalten, z. B. Alkylantien, Antimetaboliten sowie cytostatische Alkaloide, Antibiotika, Enzyme und Schwermetallverbindungen. Ferner können die Arzneimittel gegebenenfalls immunsuppressiv wirksame Substanzen und Vitamine enthalten. Die genannten Zusatzstoffe können auch in gesonderten pharmazeutischen Zubereitungen als Kombinationspräparate zu den erfindungsgemäßen Wirkstoffen gegeben werden.

Der Wirkstoffgehalt der Arzneimittel beträgt gewöhnlich 0,01 bis 95 Gewichtsprozent, vorzugsweise 0,1 bis 85 Gewichtsprozent, bezogen auf das fertige Arzneimittel.

**Patentansprüche** für die Vertragsstaaten: BE, CH, DE, FR, GB, IT, LI, LU, NL, SE

1. Metalliceniumsalze der allgemeinen Formel I

$$[(\eta^5\text{-}C_5H_{5\text{-}x}R_x)M(\eta^5\text{-}C_5H_{5\text{-}y}R'_y)]^{m+}{}_a[A]^{n-}{}_b \qquad (I)$$

in der M Fe, Co oder Ru ist;

$C_5H_{5\text{-}x}R_x$ und $C_5H_{5\text{-}y}R'_y$ Cyclopentadienyl-Ringe bedeuten, die unabhängig voneinander unsubstituiert (x, y = 0) oder ein- bis fünffach substituiert sein können (x, y = 1, 2, 3, 4 oder 5), wobei R und R' unabhängig voneinander für gleiche oder verschiedene Substituenten stehen und Alkyl, Cycloalkyl, Hydroxyalkyl, Aminoalkyl, Halogenalkyl, Alkenyl, Aryl, Aralkyl, Ferrocenyl, Ferrocenylium Ferrocenylalkyl, Acyl, Halogenyl, Trialkylsilyl, Tricycloalkylsilyl, Triarylsilyl, Triaralkylsilyl oder Carboxyl-, Ester-, Amid- oder Hydrazid-Gruppen bedeuten oder R und R' zusammen eine Alkylen-Brücke mit 2 bis 4 Brückenatomen bilden,

wobei die Alkylgruppen 1 bis 10 Kohlenstoffatome, die Cycloalkylgruppen 3 bis 8 Kohlenstoffatome, die Alkenylgruppen 2 bis 10 Kohlenstoffatome, die Arylgruppen 6 bis 18 Kohlenstoffatome und die Acylgruppen bis zu 12 Kohlenstoffatome enthalten;

a, b, m und n ganze Zahlen sind, wobei m den Wert 1 oder 2 und n den Wert 1, 2 oder 3 hat und $a \cdot m = b \cdot n$; und A ein komplexbildendes oder stark raumerfüllendes, das Metallicenium-Kation stabilisierendes Anion ist; und Solvate dieser Salze zur Verwendung als Arzneistoffe.

2. Verbindungen nach Anspruch 1, dadurch gekennzeichnet, daß M Fe oder Co ist.

3. Verbindungen nach Anspruch 1 oder 2, dadurch gekennzeichnet, daß x und y = 0.

4. Verbindungen nach einem der Ansprüche 1 bis 3, dadurch gekennzeichnet, daß $[A]^{n-}$ $BF_4^-$, $B\emptyset_4^-$, $FeCl_4^-$, $FeBr_4^-$, $BiCl_4^-$, $GaCl_4^-$, $PF_6^-$, $RuCl_4^-$ oder $(Cl_3Fe\text{-}O\text{-}FeCl_3)^2$ ist.

5. Verbindungen nach einem der Ansprüche 1 bis 3, dadurch gekennzeichnet, daß A ein Chinonid oder Chinondimethanid, ein Polyhalogenid, Vanadat, Oxyvanadat, Molybdat, Wolframat, Heteropolyanion, Reineckat oder das Anion einer starken organischen Säure bedeutet.

6. Verbindungen nach Anspruch 5, dadurch gekennzeichnet, daß A ein Mono-, Di- oder Trihalogenacetat-, Cyanacetat- oder Pikrat-Anion ist und das Salz gegebenenfalls mit freier Säure solvatisiert ist.

7. Ferricenium-trichloracetat-mono- und -bis(trichloressigsäure).

8. Diferricenium-µ-oxo-bis(trichloroferrat).

9. Ferricenium-pikrat und Ferricenium-pikrat-pikrinsäure.

10. Verwendung der Verbindungen nach einem der Ansprüche 1 bis 9 zur Herstellung eines cytostatisch wirksamen Arzneimittels.

11. Arzneimittel, enthaltend mindestens eine Verbindung nach den Ansprüchen 1 bis 9 und pharmazeutisch verträgliche Träger-, Verdünnungs- und/oder Hilfsstoffe.

**Patentansprüche** für den Vertragsstaat: MT

1. Verwendung von Metalliceniumsalzen der allgemeinen Formel I

$$[(\eta^5\text{-}C_5H_{5\text{-}x}R_x)M(\eta^5\text{-}C_5H_{5\text{-}y}R'_y)]^{m+}{}_a[A]^{n-}{}_b \qquad (I)$$

in der M Fe, Co oder Ru ist;

$C_5H_{5\text{-}x}R_x$ und $C_5H_{5\text{-}y}R'_y$ Cyclopentadienyl-Ringe bedeuten, die unabhängig voneinander unsubstituiert (x, y = 0) oder ein- bis fünffach substituiert sein können (x, y = 1, 2, 3, 4 oder 5), wobei R und R' unabhängig voneinander für gleiche oder verschiedene Substituenten stehen und Alkyl, Cycloalkyl, Hydroxyalkyl, Aminoalkyl, Halogenalkyl, Alkenyl, Aryl, Aralkyl, Ferrocenylium Ferrocenylalkyl, Acyl, Halogenyl, Trialkylsilyl, Tricycloalkylsilyl, Triarylsilyl, Triaralkylsilyl oder Carboxyl-,

Ester-, Amid- oder Hydrazid-Gruppen bedeuten oder R und R' zusammen eine Alkylen-Brücke mit 2 bis 4 Brückenatomen bilden,

wobei die Alkylgruppen 1 bis 10 Kohlenstoffatome, die Cycloalkylgruppen 3 bis 8 Kohlenstoffatome, die Alkenylgruppen 2 bis 10 Kohlenstoffatome, die Arylgruppen 6 bis 18 Kohlenstoffatome und die Acylgruppen bis zu 12 Kohlenstoffatome enthalten;

a, b, m und n ganze Zahlen sind, wobei m den Wert 1 oder 2 und n den Wert 1, 2 oder 3 hat und $a \cdot m = b \cdot n$; und A ein komplexbildendes oder stark raumerfüllendes, das Metallicenium-Kation stabilisierendes Anion ist; und Solvate dieser Salze zur Herstellung von Arzneimitteln, insbesondere cytostatisch wirksamen Arzneimitteln.

2. Verwendung nach Anspruch 1, dadurch gekennzeichnet, daß $[A]^{n-}$ $BF_4^-$, $Bø_4^-$, $FeCl_4^-$, $BiCl_4^-$, $GaCl_4^-$, $PF_6^-$, $RuCl_4^-$ oder $(Cl_3Fe\text{-}O\text{-}FeCl_3)^{2-}$ ist.

3. Verwendung nach Anspruch 1 dadurch gekennzeichnet, daß A ein Chinonid oder Chinondimethanid, ein Polyhalogenid, Vanadat, Oxyvanadat, Molybdat, Wolframat, Heteropolyanion, Reineckat oder das Anion einer starken organischen Säure bedeutet.

4. Verwendung nach Anspruch 3, dadurch gekennzeichnet, daß A ein Mono-, Di- oder Trihalogenacetat-, Cyanacetat- oder Pikrat-Anion ist und das Salz gegebenenfalls mit freier Säure solvatisiert ist.

5. Verfahren zur Herstellung von Ferricenium-tetrachloroferrat, dadurch gekennzeichnet, daß man Ferrocen und Eisen(III)-chlorid in einem Molverhältnis von 1 : 2 in Ether umsetzt, gegebenenfalls das erhaltene rohe Produkt aus absolutem Ethanol umkristallisiert, und entweder das rohe Produkt oder das aus Ethanol umkristallisierte Produkt aus einem sauren Chloridionen-haltigen Medium umkristallisiert.

6. Verfahren nach Anspruch 5, dadurch gekennzeichnet, daß man als Chloridionen-haltiges Medium Thionylchlorid oder eine Lösung von Chlorwasserstoff in wässrigem Methanol verwendet.

7. Verfahren zur Herstellung von Diferricenium-µ-oxo-bis(trichloroferrat), dadurch gekennzeichnet, daß man Ferricenium-tetrachloroferrat aus einer Mischung von Methanol und Acetonitril, die mit Pyridin versetzt wird, umkristallisiert.

8. Verfahren zur Herstellung von Ferricenium-trichloracetat-bis(trichloressigsäure), dadurch gekennzeichnet, daß man Ferrocen und Trichloressigsäure in einem Molverhältnis von 1 : 3 in Benzollösung umsetzt.

9. Verfahren zur Herstellung von Ferricenium-trichloracetat-mono(trichloressigsäure), dadurch gekennzeichnet, daß man Ferricenium-trichloracetat-bis(trichloressigsäure) in der Wärme aus Wasser umkristallisiert.

10. Verfahren zur Herstellung von Ferricenium-pikrat-pikrinsäure dadurch gekennzeichnet, daß man Ferrocen in konzentrierter Schwefelsäure oxidiert und die erhaltene, mit Wasser verdünnte Ferriceniumsulfat-Lösung mit Pikrinsäure behandelt.

**Claims** for the Contacting States: BE, CH, DE, FR, GB, IT, LI, LU, NL, SE

1. Metallicenium salts of the general formula I

$$[(\eta^5\text{-}C_5H_{5\text{-}x}R_x)M(\eta^5\text{-}C_5H_{5\text{-}y}R'_y)]^{m+}{}_a[A]^{n-}{}_b \quad (I)$$

in which M is Fe, Co or Ru;

$C_5H_{5\text{-}x}R_x$ and $C_5H_{5\text{-}y}R'_y$ denote cyclopentadienyl rings which, independently of one another, can be unsubstituted (x, y = 0) or mono- to penta-substituted (x, y = 1, 2, 3, 4 or 5), in which R and R' independently of one another represent identical or different substituents and denote alkyl, cycloalkyl, hydroxyalkyl, aminoalkyl, haloalkyl, alkenyl, aryl, aralkyl, ferrocenyl, ferrocenylium, ferrocenylalkyl, acyl, halo, trialkylsilyl, tricycloalkylsilyl, triarylsilyl, triaralkylsilyl or carboxyl, ester, amide or hydrazide groups, or R and R' together form an alkylene bridge with 2 to 4 bridge atoms, the alkyl groups containing 1 to 10 carbon atoms, the cycloalkyl groups containing 3 to 8 carbon atoms, the alkenyl groups containing 2 to 10 carbon atoms, the aryl groups containing 6 to 18 carbon atoms and the acyl groups containing up to 12 carbon atoms; a, b, m and n are integers, and m has the value 1 or 2, n has the value 1, 2 or 3 and $a \cdot m = b \cdot n$; and A is a complexing or very bulky anion which stabilizes the metallicenium cation; and solvates of these salts for use as medicaments.

2. Compounds according to claim 1, characterized in that M is Fe or Co.

3. Compounds according to claim 1 or 2, characterized in that x and y = 0.

4. Compounds according to one of claims 1 to 3, characterized in that $[A]^{n-}$ is $BF_4^-$, $Bø_4^-$, $FeCl_4^-$, $FeBr_4^-$, $BiCl_4^-$, $GaCl_4^-$, $PF_6^-$, $RuCl_4^-$ or $(Cl_3Fe\text{-}O\text{-}FeCl_3)^{2-}$.

5. Compounds according to one of claims 1 to 3, characterized in that A is a quinonide or quinonedimethanide, a polyhalide, vanadate, oxyvanadate, molybdate, tungstate, heteropolyanion or reineckate or the anion of a strong organic acid.

6. Compounds according to claim 5, characterized in that A is a mono-, di- or tri-haloacetate, cyanoacetate or picrate anion and the salt is optionally solvated with free acid.

7. Ferricenium trichloroacetate mono- and bis-(trichloroacetic acid).

8. Diferricenium μ-oxo-bis-(trichloroferrate).

9. Ferricenium picrate and ferricenium picrate-picric acid.

10. Use of the compounds according to one of claims 1 to 9 for the preparation of a cytostatically active pharmaceutical composition.

11. Pharmaceutical compositions containing at least one compound according to claims 1 to 9 and pharmaceutically acceptable excipients, diluents and/or auxiliaries.

**Claims** for the Contracting State: AT

1. Use of metallicenium salts of the general formula I

$$[(\eta^5\text{-}C_5H_{5\text{-}x}R_x)M(\eta^5\text{-}C_5H_{5\text{-}y}R'_y)]^{m+}{}_a[A]^{n-}{}_b \quad (I)$$

in which M is Fe, Co or Ru;
$C_5H_{5\text{-}x}R_x$ and $C_5H_{5\text{-}y}R'_y$ denote cyclopentadienyl rings which, independently of one another, can be unsubstituted (x, y = 0) or mono- to penta-substituted (x, y = 1, 2, 3, 4 or 5), in which R and R' independently of one another represent identical or different substituents and denote alkyl, cycloalkyl, hydroxyalkyl, aminoalkyl, haloalkyl, alkenyl, aryl, aralkyl, ferrocenyl, ferrocenylium, ferrocenylalkyl, acyl, halo, trialkylsilyl, tricycloalkylsilyl, triarylsilyl, triaralkylsilyl or carboxyl, ester, amide or hydrazide groups, or R and R' together form an alkylene bridge with 2 to 4 bridge atoms, the alkyl groups containing 1 to 10 carbon atoms, the cycloalkyl groups containing 3 to 8 carbon atoms, the alkenyl groups containing 2 to 10 carbon atoms, the aryl groups containing 6 to 18 carbon atoms and the acyl groups containing up to 12 carbon atoms; a, b, m and n are integers, and m has the value 1 or 2, n has the value 1, 2 or 3 and $a \cdot m = b \cdot n$; and A is a complexing or very bulky anion which stabilizes the metallicenium cation; and solvates of these salts, for the preparation of medicaments, in particular cytostatically active medicaments.

2. Use according to claim 1, characterized in that $[A]^{n-}$ is $BF_4^-$, $B\text{ø}_4^-$, $FeCl_4^-$, $FeBr_4^-$, $BiCl_4^-$, $GaCl_4^-$, $PF_6^-$, $RuCl_4$ or $(Cl_3Fe\text{-}O\text{-}FeCl_3)^{2-}$.

3. Use according to claim 1, characterized in that A is a quinonide or quinonedimethanide, a polyhalide, vanadate, oxyvanadate, molybdate, tungstate, heteropolyanion or reineckate or the anion of a strong organic acid.

4. Use according to claim 3, characterized in that A is a mono-, di or tri-haloacetate, cyanoacetate or picrate anion and the salt is optionally solvated with free acid.

5. Process for preparation of ferricenium tetrachloroferrate, characterized in that ferrocene and iron (III) chloride are reacted in a molar ratio of 1 : 2 in ether, the crude product obtained is recrystallized, if desired, from absolute ethanol, and either the crude product or the product recrystallized from ethanol is recrystallized from an acidic medium containing chloride ions.

6. Process according to claim 5, characterized in that the medium containing chloride ions which is used is thionyl chloride or a solution of hydrogen chloride in aqueous methanol.

7. Process for the preparation of diferricenium μ-oxo-bis-(trichloroferrate), characterized in that ferricenium tetrachloroferrate is recrystallized from a mixture of methanol and acetonitrile to which pyridine is added.

8. Process for the preparation of ferricenium trichloroacetate bis-(trichloroacetic acid), characterized in that ferrocene and trichloroacetic acid are reacted in a molar ratio of 1 : 3 in benzene solution.

9. Process for the preparation of ferricenium trichloroacetate mono-(trichloroacetic acid), characterized in that ferricenium trichloroacetate bis-(trichloroacetic acid) is recrystallized from warm water.

10. Process for the preparation of ferricenium picrate-picric acid, characterized in that ferrocene is oxidized in concentrated sulphuric acid, and the ferricenium sulphate solution obtained is diluted with water and treated with picric acid.

**Revendications** pour les Etats Contractants: BE, CH, DE, FR, GB, IT, LI, LU, NL, SE

1. Sels de métallicénium, de formule générale I

$$[(\eta^5\text{-}C_5H_{5\text{-}x}R_x)M(\eta^5\text{-}C_5H_{5\text{-}y}R'_y)]^{m+}{}_a\,[A]^{n-}{}_b \qquad (I)$$

dans laquelle M est Fe, Co ou Ru; $C_5H_{5\text{-}x}R_x$ et $C_5H_{5\text{-}y}R'_y$ représentent des noyaux cyclopentadiényle, qui, indépendamment l'un de l'autre, peuvent chacun être non-substitués (x, y = 0) ou substitués une à cinq fois (x, y = 1, 2, 3, 4 ou 5), R et R', indépendamment l'un de l'autre, représentant chacun des substituants identiques ou différents et sont chacun un radical alkyle, cycloalkyle, hydroxyalkyle, aminoalkyle, halogènealkyle, alcényle, aryle, aralkyle, ferrocényle, ferrocénylium, ferrocénylalkyle, acyle, halogényle, trialkylsilyle, tricycloalkylsilyle, triarylsilyle, triaralkylsilyle ou carboxyle, ester, amide ou hydrazide, ou encore R et R' formant ensemble un enchaînement alkylène ayant de 2 à 4 atomes pontants, où les groupes alkyle contiennent de 1 à 10 atomes de carbone, les groupes cycloalkyle de 3 à 8 atomes de carbone, les groupes alcényle de 2 à 10 atomes de carbone, les groupes aryle de 6 à 18 atomes de carbone et les groupes acyle jusqu'à 12 atomes de carbone; a, b, m et n sont des nombres entiers, m ayant la valeur 1 ou 2, et n la valeur 1, 2 ou 3, et $a \cdot m = b \cdot n$; et A est un anion complexant ou fortement encombrant, qui stabilise le cation métallicénium; et des produits de solvatation de ces sels, pour utilisation en tant que médicaments.

2. Composés selon la revendication 1, caractérisés en ce que M est Fe ou Co.

3. Composés selon la revendication 1 ou 2, caractérisés en ce que x et y = 0.

4. Composés selon l'une des revendications 1 à 3, caractérisés en ce que $[A]^{n-}$ est $BF_4^-$, $B\varnothing_4^-$, $FeCl_4^-$ $FeBr_4^-$, $BiCl_4^-$, $GaCl_4^-$, $PF_6^-$, $RuCl_4^-$ ou $(Cl_3Fe\text{-}O\text{-}FeCl_3)^{2-}$.

5. Composés selon l'une des revendications 1 à 3, caractérisés en ce que A est un quinonure ou un quinonediméthanure, un polyhalogénure, un vanadate, un oxyvanadate, un molybdate, un tungstate, un hétéropolyanion, un reineckate ou l'anion d'un acide organique fort.

6. Composés selon la revendication 5, caractérisés en ce que A est un anion mono-, di- ou trihalogénoacétate, cyanoacétate ou picrate, et le sel est éventuellement solvaté avec un acide libre.

7. Trichloroacétats de ferricénium/mono- et bis(acide trichloracétique).

8. µ-oxo-bis(trichloroferrate) de diférricénium.

9. Picrate de ferricénium, et picrate de ferricénium / acide picrique.

10. Utilisation des composés selon l'une des revendications 1 à 9 pour la préparation d'un médicament à effet cytostatique.

11. Médicament contenant au moins un composé selon les revendications 1 à 9 et des excipients, diluants et/ou adjuvants pharmaceutiquement tolérables.

**Revendications** pour l'Etat Contractant: AT

1. Utilisation de sels de métallicénium, de formule générale I

$$[\eta^5\text{-}C_5H_{5\text{-}x}R_x)M(\eta^5\text{-}C_5H_{5\text{-}y}R'_y)]^{m+}{}_a\,[A]^{n-}{}_b \qquad (I)$$

dans laquelle M est Fe, co ou Ru; $C_5H_{5\text{-}x}R_x$ et $C_5H_{5\text{-}y}R'_y$ représentent des noyaux cyclopentadiényle, qui, indépendamment l'un de l'autre, peuvent chacun être non substitués (x, y = 0) ou substitués une à cinq fois (x, y = 1, 2, 3, 4 ou 5), R et R', indépendamment l'un de l'autre, représentant chacun des substituants identiques ou différents et sont chacun un radical alkyle, cycloalkyle, hydroxyalkyle, aminoalkyle, halogènealkyle, alcényle, aryle, aralkyle, ferrocényle, ferrocénylium, ferrocénylalkyle, acyle, halogényle, trialkylsilyle, tricycloalkylsilyle, triarylsilyle, triaralkylsilyle ou carboxyle, ester, amide ou hydrazide, ou encore R et R' formant ensemble un enchaînement alkylène ayant de 2 à 4 atomes pontants, où les groupes alkyle contiennent de 1 à 10 atomes de carbone, les groupes cycloalkyle de 3 à 8 atomes de carbone, les groupes alcényle de 2 à 10 atomes de carbone, les groupes aryle de 6 à 18 atomes de carbone et les groupes acyle jusqu'à 12 atomes de carbone; a, b, m et n sont des nombres entiers, m ayant la valeur 1 ou 2, et n la valeur 1, 2 ou 3, et $a \cdot m = b \cdot n$; et A est un anion complexant ou fortement encombrant, qui stabilise le cation métallicénium; et des produits de solvatation de ces sels, pour la préparation de médicaments, en particulier de médicaments à effet cytostatique.

2. Utilisation selon la revendication 1, caractérisée en ce que $[A]^{n-}$ est $BF_4-$, $Bø_4-$, $FeCl_4-$, $FeBr_4-$, $BiCl_4-$, $GaCl_4-$, $PF_6-$, $RuCl_4-$ ou $(Cl_3Fe\text{-}O\text{-}FeCl_3)^{2-}$.

3. Utilisation selon la revendication 1, caractérisée en ce que A est un quinonure ou un quinonediméthanure, un polyhalogénure, un vanadate, un oxyvanadate, un molybdate, un tungstate, un hétéropolyanion, un reineckate ou l'anion d'un acide organique fort.

4. Utilisation selon la revendication 3, caractérisée en ce que A est un anion mono-, di- ou trihalogénoacétate, cyanoacétate ou picrate, et le sel est éventuellement solvaté avec un acide libre.

5. Procédé de préparation de tétrachloroferrate de ferricénium, caractérisé en ce qu'on fait réagir, dans de l'éther, du ferrocène et du chlorure de fer(III), selon un rapport en moles de 1 : 2, on recristallise éventuellement, dans de l'éthanol absolu, le produit brut obtenu, et on recristallise le produit brut ou le produit recristallisé dans l'éthanol, dans un milieu contenant des ions chlorure acides.

6. Procédé selon la revendication 5, caractérisé en ce qu'on utilise, comme milieu contenant des ions chlorure, du chlorure de thionyle ou une solution d'acide chlorhydrique dans du méthanol aqueux.

7. Procédé de préparation de µ-oxo-bis(trichloroferrate) de diferricénium, caractérisé en ce qu'on recristallise du tétrachloroferrate de ferricénium dans un mélange de méthanol et d'acétonitrile additionné de pyridine.

8. Procédé de préparation de trichloroacétate de ferricénium / bis(acide trichloroacétique), caractérisé en ce qu'on fait réagir du ferrocène et de l'acide trichloroacétique dans une solution benzénique, selon un rapport en moles de 1 : 3.

9. Procédé de préparation de trichloroacétate de ferricénium / mono(acide trichloroacétique), caractérisé en ce qu'on recristallise le trichloroacétate de ferricénium / bis(acide trichloroacétique) à chaud dans de l'eau.

10. Procédé de préparation de picrate de ferricénium / acide picrique, caractérisé en ce qu'on oxyde du ferrocène dans de l'acide sulfurique concentré et qu'on traite à l'acide picrique la solution obtenue de sulfate de ferricénium diluée à l'eau.